(19) Europäisches Patentamt
European Patent Office
Office européen des brevets

(11) **EP 1 234 574 B1**

(12) **EUROPEAN PATENT SPECIFICATION**

(45) Date of publication and mention
of the grant of the patent:
**26.11.2008 Bulletin 2008/48**

(51) Int Cl.:
*A61K 31/185* (2006.01)    *A61K 8/46* (2006.01)
*A61Q 7/00* (2006.01)    *A61Q 5/02* (2006.01)
*A61Q 5/12* (2006.01)    *A61P 43/00* (2006.01)
*A61P 17/14* (2006.01)    *A61P 17/16* (2006.01)

(21) Application number: **01967782.2**

(22) Date of filing: **21.09.2001**

(86) International application number:
**PCT/JP2001/008243**

(87) International publication number:
**WO 2002/034253 (02.05.2002 Gazette 2002/18)**

(54) **HAIR GROWTH STIMULANT**

HAARWUCHSMITTEL

STIMULANT DE LA CROISSANCE DES CHEVEUX

(84) Designated Contracting States:
**DE FR GB IT**

(30) Priority: **26.10.2000  JP 2000326467**
**06.11.2000  JP 2000337029**

(43) Date of publication of application:
**28.08.2002  Bulletin 2002/35**

(73) Proprietor: **SHISEIDO COMPANY LIMITED**
**Chuo-ku,**
**Tokyo 104-8010 (JP)**

(72) Inventors:
• **HAMADA, Chika**
**c/o SHISEIDO RESEARCH CENTER**
**Yokohama-shi, Kanagawa 224-8558 (JP)**
• **TAKAHASHI, Tadahito**
**c/o SHISEIDO RESEARCH CENTER**
**Yokohama-shi, Kanagawa 224-8558 (JP)**
• **TAJIMA, Masahiro**
**c/o SHISEIDO RESEARCH CENTER**
**Yokohama-shi, Kanagawa 224-8558 (JP)**
• **NAKAMA, Yasunari**
**c/o SHISEIDO RESEARCH CENTER**
**Yokohama-shi, Kanagawa 224-8558 (JP)**
• **MAGARA, Tsunao**
**c/o SHISEIDO RESEARCH CENTER**
**Yokohama-shi, Kanagawa 224-8558 (JP)**

(74) Representative: **TER MEER - STEINMEISTER &**
**PARTNER GbR**
**Patentanwälte**
**Mauerkircherstrasse 45**
**81679 München (DE)**

(56) References cited:
EP-A2- 0 286 261          CH-A- 366 831
DE-B- 1 248 865          JP-A- 1 199 961
JP-A- 5 310 549          JP-A- 11 292 753
JP-A- 58 065 214          JP-A- 2001 019 622
US-A- 3 636 195          US-A- 3 745 181

• **DATABASE BIOSIS [Online] BIOSCIENCES INFORMATION SERVICE, PHILADELPHIA, PA, US; June 2003 (2003-06), MAGARA T ET AL: "The effect of taurine and its derivatives on the hair follicle cell." XP002350791 Database accession no. PREV200400184826 -& JOURNAL OF INVESTIGATIVE DERMATOLOGY SYMPOSIUM PROCEEDINGS, vol. 8, no. 1, June 2003 (2003-06), page 140, XP002350793 THIRD INTERCONTINENTAL MEETING OF HAIR RESEARCH SOCIETIES; TOKYO, JAPAN; JUNE 13-15, 2001 ISSN: 1087-0024**

**Description**

TECHNICAL FIELD

The present invention relates to a hair fostering shampoo or rinse.

BACKGROUND ART

In today's society, namely an aging society and a stressed society, there are various factors causing hair loss. Research and development into superior hair growth agents is thus being carried out with vigor.

The following are examples of effects of hair growth agents on hair: hair growth inducing effects (hair growth promoting effects, growth period inducing effects), hair thickening effects, hair growth period extending effects, $5\alpha$-reductase inhibiting effects, blood circulation promoting effects, bactericidal effects, dandruff preventing effects, moisture retaining effects, antioxidative effects.

However, despite the vigorous research and development into hair growth agents, the hair fostering action, namely hair loss prevention and hair growth effects and the like, of conventional hair growth agents has not always been adequate. This is because there are various causes of hair loss, and moreover the mechanism of hair growth is extremely complex.

Conventional hair growth agents have been developed considering only the phenomena of 'hair growth' and 'hair loss'; there are few hair growth agents that have been developed by delving into the mechanisms thereof.

One large reason for this is that hair fostering medicament test methods allowing easy verification of hair fostering effects focusing on the mechanism thereof had not been adequately established. In particular, it is difficult to establish a hair fostering medicament test method for verifying hair growth period extending effects. As a result, many conventional hair growth agents have been developed focusing not on growth period extending effects, but rather on hair growth inducing effects in which hair is induced during the hair growth period in the hair cycle.

The present inventors thus established a simple hair fostering medicament test method for testing for hair growth period extending effects that is carried out in vitro, and using this hair fostering medicament test method, discovered a component having hair growth period extending effects. Moreover, the present inventors discovered unexpected effects in that this component gives hair fitness and body and gives hair a feeling of volume, thus accomplishing the present invention.

Object of the present invention is as follows. To provide a hair growth agent (a hair fostering shampoo rinse) that has hair growth period extending effects, and moreover is capable of giving hair fitness and body and giving hair a feeling of volume.

DISCLOSURE OF INVENTION

The present invention is as follows. A hair fostering shampoo or rinse containing N-methyltaurine.

BRIEF DESCRIPTION OF DRAWINGS

Fig. 1 is a graph showing extent of proliferation of outer hair root sheath cells.
Fig. 2 is a graph showing increase in torsional torque of hair.

BEST MODE FOR CARRYING OUT THE INVENTION

Following is a detailed description of the present invention.

N-methyltaurine, which is used in the present invention, is a compound represented by the molecular formula $HNCH_3CH_2CH_2SO_3H$. Up until now, the hair growth period extending effects and effects of giving fitness and body to hair of N-methyltaurine have not been verified, and N-methyltaurine has not been used in a hair growth agent.

In the present invention, 'hair growth agent' refers to a hair-related product for the purpose of fostering hair being a shampoo or rinse.

According to the hair fostering medicament test method described below, the hair growth agent of the present invention has an effect of maintaining or extending the hair growth period by maintaining or promoting the division growth activity of at least hair follicle epithelial cells.

The hair fostering shampoo of the present invention is a preferable form in which the above-mentioned hair growth agent is used with cleaning components (surfactants etc.) mixed therein as a hair-fostering hair-cleaning agent.

The hair growth agent of the present invention is particularly effective in the case of alopecia caused by the growth period becoming short due to proliferation of hair follicle epithelial cells around the hair root being sluggish, and thus the proportion of resting period hairs relative to growth period hairs becoming high.

By using the hair growth agent of the present invention in combination with a hair fostering medicament having another individual effect, it is possible to achieve a combined or synergistic effect in the case of alopecia.

The amount of N-methyltaurine mixed into the hair growth agent is determined as appropriate in accordance with the specific form of the hair growth agent, such that the effects of the present invention will be exhibited.

The N-methyltaurine content is generally 0.00001 to 20 mass%, preferably 0.01 to 10.0 mass%, relative to the total amount of the hair growth agent.

If the N-methyltaurine content is less than 0.00001 mass%, then the hair growth period extending effect based on the hair follicle cell proliferation activating action or the outer hair root sheath cell proliferation activating action will be insufficient.

If the N-methyltaurine content is more than 20 mass%, then it will not be possible to expect an increase in the effects commensurate with the increase in the N-methyltaurine content. Moreover the tendency for there to be difficulties in preparing the desired pharmaceutical form will become marked.

The hair growth agent of the present invention has a hair growth period extending effect based on an excellent hair follicle cell proliferation activating action or outer hair root sheath cell proliferation activating action.

The hair growth agent of the present invention is thus particularly effective in the case of alopecia caused by the growth period becoming short due to proliferation of hair follicle epithelial cells around the hair root being sluggish, and as a result the proportion of resting period hairs relative to growth period hairs becoming high.

Moreover, by using N-methyltaurine and a hair fostering component having another individual effect in combination, it is possible to achieve a synergistic hair fostering effect.

In the present invention, there are no limitations on the means for verifying the hair growth period extending effect (maintenance or extension of the growth period in the hair cycle). An in vitro characterization method or an in vivo characterization method can be used. Considering convenience and effectiveness, however, it is preferable to use an in vitro characterization method.

Following is a brief description of a method of characterizing, in vitro, the cultured hair follicle epithelial cell proliferation effect in a hair growth period extending effect.

In this method, an object substance is made to come into contact with cultured hair follicle epithelial cells in a serum-free medium, and thus the presence/absence or strength/weakness of the proliferation activity on the cells is characterized.

The characterization method is a hair fostering medicament test method in which the effects of extending the growth period in the hair cycle are evaluated.

That is, it is an in vitro hair fostering medicament test method in which growth period extending effects in the hair cycle are characterized by focussing on hair follicle epithelial cells, which are directly related to hair elongation, and using cultured cells thereof.

In this hair fostering medicament test method, an object substance is made to come into contact with 'cultured hair follicle epithelial cells', which are cultured cells obtained by isolating hair follicle epithelial cells from an animal (including humans), and then the presence/absence or strength/weakness of the proliferation of these cultured cells is characterized.

Hair follicle epithelial cells' refers to cells such as matrix cells and outer hair root sheath cells in the vicinity of the hair root in particular, with hair papilla cells on the inside being excluded.

The growth period in the hair cycle is the period when the hair elongates (the period when the hair follicle epithelial cells divide and proliferate). When this slows down, a regressive period and a resting period are entered, and hair growth stops.

A substance capable of extending the growth period in the hair cycle is thus capable of maintaining division and proliferation activity of the hair follicle epithelial cells. As a result, transition to the regressive period and the resting period in the hair cycle is prevented, and hair loss is prevented.

The hair growth agent of the present invention thus has an effect of promoting or maintaining hair follicle epithelial cell proliferation.

Note that another in vitro hair fostering medicament test method is, for example, a method in which an object substance is made to act on hair papilla cells from an animal, and the proliferation effects are then judged.

An example of a method of characterizing hair follicle epithelial cell proliferation effects in vivo is a method in which the object substance is administered to, for example, a nude mouse, and the state of hair growth sites on the body surface of the nude mouse is characterized, and hence the effects of the object substance in extending the growth period in the hair cycle are examined.

That is, this is a hair fostering medicament test method in which a nude mouse, which is as a rule hairless but for which distinctive hair growth occurs in which hair growth sites shift on the body surface with time, is used, and by characterizing the broadness of the hair growth sites and the rate of shifting of the hair growth sites, the length of the growth period in the hair cycle is examined.

There are no particular limitations on the pharmaceutical form of the hair growth agent of the present invention, provided that application to the scalp and head hair is possible. For example, a liquid, a milky lotion, an ointment or the like can be selected.

Product forms of the hair growth agent are hair fostering shampoos and hair fostering rinses. By using a hair fostering shampoo containing cleaning components (for example, surfactants) day after day, the hair fostering effects are readily exhibited, and moreover the hair is given fitness and body, and the hair can be given a feeling of volume.

Components generally used in cosmetics, non-medicinal products and medicines can be blended into the hair growth agent of the present invention as required, so long as this is within a range such that the effects of the present invention are not impaired, and preparation can be carried out using conventional methods. Such generally used components are, for example, powder components, liquid oils, solid fats, waxes, hydrocarbon oils, higher fatty acids, higher alcohols, ester oils, silicone oils, anionic surfactants, cationic surfactants, ampholytic surfactants, nonionic surfactants, moisturizing agents, water-soluble macromolecules, thickeners, film-forming agents, UV absorbers, metal ion sequestering agents, lower alcohols, polyhydric alcohols, sugars, amino acids, organic amines, macromolecule emulsions, pH regulators, skin nutrients, vitamins, antioxidants, antioxidant auxiliaries, aromatics, and water.

Specific examples of components that can be blended in are as follows. One type or two or more types of the following components are blended in as necessary, and manufacturing is carried out using conventional methods in accordance with the desired pharmaceutical form.

Inorganic powders:

Talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, metal salts of tungstic acid, magnesium, silica, zeolite, barium sulfate, baked calcium sulfate (calcined gypsum), calcium phosphate, fluoroapatite, hydroxyapatite, ceramic powders, metal soaps (zinc myristate, calcium palmitate, aluminum stearate), boron nitride.

Organic powders:

Polyamide resin powders (nylon powder), polyethylene powder, polymethyl methacrylate powder, polystyrene powder, styrene / acrylic acid copolymer resin powder, benzoguanamine resin powder, polytetrafluoroethylene powder, cellulose powder.

Inorganic pigments:

Inorganic white pigments (titanium dioxide, zinc oxide), inorganic red pigments (iron oxide (burnt ocher), iron titanate), inorganic brown pigments (γ-iron oxide), inorganic yellow pigments (yellow iron oxide, loess), inorganic black pigments (black iron oxide, low oxides of titanium), inorganic purple pigments (mango violet, cobalt violet), inorganic green pigments (chromium oxide, chromium hydroxide, cobalt titanate), inorganic blue pigments (ultramarine, iron blue); pearl pigments (titanium-oxide-coated mica, titanium-oxide-coated bismuth oxychloride, titanium-oxide-coated talc, colored titanium-oxide-coated mica, bismuth oxychloride, fish scale flake); metal powder pigments (aluminum powder, copper powder).

Organic pigments:

Red 201, red 202, red 204, red 205, red 220, red 226, red 228, red 405, orange 203, orange 204, yellow 205, yellow 401, blue 404, red 3, red 104, red 106, red 227, red 230, red 401, red 505, orange 205, yellow 4, yellow 5, yellow 202, yellow 203, green 3, blue 1 ; natural pigments (chlorophyll, β-carotene).

Liquid oils:

Avocado oil, camellia oil, turtle oil, macadamia nut oil, corn oil, mink oil, olive oil, rapeseed oil, egg yolk oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, china wood oil, Japanese paulownia oil, jojoba oil, germ oil, triglycerol.

Solid fats:

Cacao butter, coconut oil, horse fat, hardened coconut oil, palm oil, beef tallow, mutton tallow, hardened beef tallow, palm kernel oil, lard, beef bone fat, sumac kernel oil, hardened oils, nest's-foot oil, sumac wax, hardened castor oil.

Waxes:

Beeswax, candelilla wax, cotton wax, carnauba wax, bay berry wax, Chinese wax, spermaceti wax, montan wax, bran wax, lanolin, kapok wax, lanolin acetate, liquid lanolin, sugarcane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, polyethylene glycol lanolin fatty acid, POE hydrogenated lanolin alcohol ether.

Hydrocarbon oils:

Liquid paraffin, ozokerite, squalane, pristane, paraffin, ceresin, squalene, vaseline, microcrystalline wax.

Higher fatty acids:

Lauric acid, myristic acid, palmitic acid, stearic acid, behenic acid, oleic acid, undecylenic acid, tall acid, isostearic acid, linoleic acid, linolenic acid, eicosapentaenoic acid (EPA), docosahexaenoic acid (DHA).

Higher alcohols:

Linear alcohols (lauryl alcohol, cetyl alcohol, stearyl alcohol, behenyl alcohol, myristyl alcohol, oleyl alcohol, ceto-stearyl alcohol), branched-chain alcohols (batyl alcohol, 2-decyltetradecynol, lanolin alcohol, cholesterol, phytosterol, hexyldodecanol, isostearyl alcohol, octyldodecanol).

Ester oils:

Isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid esters, N-alkyl glycol monoisostearates, neopentyl glycol dicaprate, diisostearyl malate, glyceryl di-2-heptylundecanoate, trimethylol propane tri-2-ethylhexanoate, trimethylol propane triisostearate, pentaerythritol tetra-2-ethylhexanoate, glyceryl tri-2-ethylhexanoate, glyceryl trioctanoate, glyceryl triisopalmitate, trimethylol propane triisostearate, cetyl 2-ethylhexanoate, 2-ethylhexyl palmitate, glyceryl trimyristate, tri-2-heptylundecanoic acid glyceride, castor oil fatty acid methyl ester, oleyl oleate, acetoglycerides, 2-heptylundecyl palmitate, diisobutyl adipate, N-lauroyl-L-glutamic acid 2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate.

Silicone oils:

Linear polysiloxanes (dimethylpolysiloxane, methylphenylpolysiloxane, diphenylpolysiloxane), cyclic polysiloxanes (octamethylcyclotetrasiloxane, decamethylcyclopentasiloxane, dodecamethylcyclohexasiloxane), 3-D network structure silicone resins, silicone rubbers, modified polysiloxanes (amino-modified polysiloxanes, polyether-modified polysiloxanes, alkyl-modified polysiloxanes, fluorine-modified polysiloxanes).

Anionic surfactants:

Fatty acid soaps (sodium laurate, sodium palmitate), higher alkyl sulfuric acid ester salts (sodium lauryl sulfate, potassium lauryl sulfate), alkyl ether sulfuric acid ester salts (triethanolamine POE lauryl sulfate, sodium POE lauryl sulfate), N-acylsarcosinates (sodium lauroylsarcosinate), higher fatty acid amidosulfonates (N-myristoyl-N-methyltaurine sodium salt, coconut oil fatty acid methyltaurid sodium salt, lauryl methyltaurid sodium salt), phosphoric acid ester salts (sodium POE oleyl ether phosphate, POE-stearylether phosphate), sulfosuccinates (sodium di-2-ethylhexyl sulfosuccinate, sodium monolauroyl monoethanolamide polyoxyethylene sulfosuccinate, sodium lauryl polypropylene glycol sulfosuccinate), alkyl benzene sulfonates (linear sodium dodecylbenzenesulfonate, linear triethanolamine dodecylbenzenesulfonate, linear dodecyl benzene sulfonate), higher fatty acid ester sulfuric acid ester salts (sodium hardened-coconut-oil-fatty-acid glycerol sulfate), N-acyl glutamates (monosodium N-lauroylglutamate, disodium N-stearoylglutamate, monosodium N-myristoyl-L-glutamate), sulfonated oils (turkey red oil), POE-alkylether carboxylate, POE alkylarylether carboxylates, $\alpha$-olefin sulfonates, higher fatty acid ester sulfonates, secondary alcohol sulfuric acid ester salts, higher fatty acid alkylol amide sulfuric acid ester salts, sodium lauroyl monoethanolamide succinate, ditriethanolamine N-palmitoylaspartate; sodium caseinate.

Cationic surfactants:

Alkyltrimethylammonium salts (stearyltrimethylammonium chloride, lauryltrimethylammonium chloride), alkyl pyridinium salts (cetyl pyridinium chloride), distearyldimethylammonium chloride dialkyl dimethylammonium salt, poly (N, N'-dimethyl-3, 5-metyhlenepiperidinium) chloride, alkyl quaternary ammonium salts, alkyl dimethylbenzyl ammonium salts, alkyl isoquinolinium salts, dialkylmorpholine salt, POE alkylamines, alkylamine salts, polyamine fatty acid derivatives, amyl alcohol fatty acid derivatives, benzalkonium chloride, benzethonium chloride.

Ampholytic surfactants:

Imidazoline type ampholytic surfactants (2-undecyl-N,N,N-(hydroxyethylcarboxymethyl)-2-imi dazoline sodium salt, 2-cocoyl-2-imidazolinium-hydroxide-1-carboxyethylo xy disodium salt), betaine type surfactants (2-heptadecyl-N-carboxymethyl-N-hydroxyethylimidaz olinium betaine, lauryldimethylaminoacetic acid betaine, alkyl betaines, amide betaines, sulfobetaines).

Lipophilic nonionic surfactants:

Sorbitan fatty acid esters (sorbitan monooleate, sorbitan monoisostearate, sorbitan monolaurate, sorbitan monopalmitate, sorbitan monostearate, sorbitan sesquioleate, sorbitan trioleate, penta-2-ethylhexyl acid diglycerol sorbitan, tetra-2-ethylhexyl acid diglycerol sorbitan). glycerin polyglycerin fatty acid esters (glycerin monocotton seed oil fatty acid esters, glyceryl monoerucate, glyceryl sesquioleate, glyceryl monostearate, $\alpha,\alpha'$-glycerin oleate pyroglutamate, glyceryl mono stearate mono malate), propylene glycol fatty acid esters (propylene glycol monostearate), hardened castor oil derivatives, glycerol alkyl ethers.

Hydrophilic nonionic surfactants:

POE sorbitan fatty acid esters (POE sorbitan monooleate, POE sorbitan monostearate, POE-sorbitan monoolate, POE sorbitan tetraoleate), POE sorbit fatty acid esters (POE sorbit monolaurate, POE sorbit monooleate, POE sorbit pentaoleate, POE sorbit monostearate), POE glycerol fatty acid esters (POE glycerol monostearate, POE glycerol monoisostearate, POE-monooleate such as POE glycerol triisostearate), POE fatty acid esters (POE distearate, POE-monodioleate, ethylene glycol distearate), POE alkyl ethers (POE lauryl ether, POE oleyl ether, POE stearyl ether, POE behenyl ether, POE 2-octyldodecyl ether, POE cholestanol ether), PLUARONIC), POE POP alkyl ethers (POE POP cetyl ether, POE POP decyltetradecyl ether, POE POP monobutyl ether, POE POP hydrogenated lanolin, POE POP glycerol ether), Tetra-POE tetra-POP ethylenediamine condensate (tetronic), POE castor oil / hardened castor oil derivatives (POE castor oil, POE hardened castor oil, POE hardened castor oil monoisostearate, POE hardened castor oil triisostearate, POE hardened castor oil monopyroglutamic acid monoisostearic acid diester, POE-hydrogenated castor oil maleic acid ester), POE beeswax/lanolin derivatives (POE sorbit beeswax), alkanol amides (coconut oil fatty acid diethanolamide, lauric acid monoethanolamide, fatty acid isopropanolamides), POE propylene glycol fatty acid esters, POE alkylamines, POE fatty acid amides, saccharose fatty acid esters, alkylethoxydimethylamine oxide, trioleyl phosphate.

Moisturizing agents:

Polyethylene glycol, propylene glycol, glycerol, 1,3-butylene glycol, xylitol, sorbitol, maltitol, chondroitin sulfate, hyaluronic acid, mucoitin sulfate, charonin acid, atelocollagen, cholesteryl-12-hydroxystearate, sodium lactate, bile salt, d1-pyrrolidone carboxylates, short-chain soluble collagen, diglycerol (EO) PO adduct, *Rosa roxburghii* extract, yarrow extract, melilot extract.

Natural water-soluble macromolecules:

Macromolecules from plants (gum arabic, tragacanth gum, galactan, guar gum, carob gum, karaya gum, carrageenan, pectin, agar, quince seed, algae colloid (seaweed extract), starches (rice, corn, potato, wheat), glycyrrhizinic acid), macromolecules from microorganisms (xanthan gum, dextran, succinoglucan, pullulan) macromolecules from animals (collagen, casein, albumin, gelatin).

Semi-synthetic water-soluble macromolecules:

Starch type macromolecules (carboxymethyl starch, methylhydroxypropyl starch), cellulose type macromolecules (methylcellulose, ethylcellulose, methylhydroxypropylcellulose, hydroxyethylcellulose, cellulose sulfate sodium salt, hydroxypropylcellulose, carboxymethylcellulose, carboxymethylcellulose sodium, crystalline cellulose, cellulose powder), alginic acid type macromolecules (sodium alginate, alginic acid propylene glycol ester).

Synthetic water-soluble macromolecules:

Vinyl type macromolecules (polyvinyl alcohol, polyvinyl methyl ether, polyvinylpyrrolidone, carboxyvinyl polymer), polyoxyethylene type macromolecules (polyethylene glycol 20,000, 40,000 and 60,000 polyoxyethylene / polyoxypropylene copolymers), acrylic type macromolecules (sodium polyacrylate, polyethylacrylate, polyacrylamide), polyethylenimine, cationic polymers.

Thickeners:

Gum arabic, carrageenan, karaya gum, tragacanth gum, carob gum, quince seed (marumero), casein, dextrin, gelatin, sodium pectate, sodium alginate, methylcellulose, ethylcellulose, CMC, hydroxyethylcellulose, hydroxypropylcellulose, PVA, PVM, PVP, sodium polyacrylate, carboxyvinyl polymer, locust bean gum, guar gum, tamarind gum, dialkyldimethylammonium sulfate cellulose, xanthan gum, aluminum magnesium silicate, bentonite, hectorite, AlMg silicate (beagum), laponite, anhydrous silicic acid.

UV absorbers:

Benzoate type UV absorbers (para-aminobenzoic acid (hereinafter abbreviated to 'PABA'), PABA monoglycerol ester, N,N-dipropoxy-PABA ethyl ester, N,N-diethoxy-PABA ethyl ester, N,N-dimethyl-PABA ethyl ester, N,N-dimethyl-PABA butyl ester, N,N-dimethyl-PABA ethyl ester), anthranilate type UV absorbers (homomenthyl-N-acetyl anthranilate), salicylate type UV absorbers (amyl salicylate, menthyl salicylate, homomenthyl salicylate, octyl salicylate, phenyl salicylate, benzyl salicylate, p-isopropanolphenyl salicylate), cinnamate type UV absorbers (octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-$\alpha$-cyano-$\beta$-phenyl cinnamate, 2-ethylhexyl-$\alpha$-cyano-$\beta$-phenyl cinnamate, glyceryl mono-2-ethylhexanoyl-diparamethoxy cinnamate), benzophenone type UV absorbers (2,4-dihydroxybenzophenone, 2,2'-dihydroxy-4-methoxybenzophenone, 2, 2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone, 3-(4'-methylbenzylidene)-d,1-camphor, 3-benzylidene-d,1-camphor), 2-phenyl-5-methylbenzoxazole, 2,2'-hydroxy-5-methylphenylbenzotriazole, 2-(2'-hydroxy-5'-t-octylphenyl)benzotriazole, 2-(2'-hydroxy-5'-methylphenyl) benzotriazole, dibenzaladine, dianisoyl-methane, 4-methoxy-4'-t-butyldibenzoyl-methane, 5-(3,3-dimethyl-2-norbornylidene)-3-pentan-2-one).

Metal ion sequestering agents:

1-hydroxyethane-1,1-diphosphonic acid, tetrasodium 1-hydroxyethane-1,1-diphosphonate, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium ethylenediamine hydroxyethyl triacetate.

Lower alcohols:

Ethanol, propanol, isopropanol, isobutyl alcohol, t-butyl alcohol.

Polyhydric alcohols:

Dihydric alcohols (ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol), trihydric alcohols (glycerol, trimethylol propane), tetrahydric alcohols (pentaerythritol, 1,2,6-hexanetriol), pentahydric alcohols (xylitol), hexahydric alcohols (sorbitol, mannitol), polyhydric alcohol polymers (diethylene glycol, dipropylene glycol, triethylene glycol, polypropylene glycol, tetraethylene glycol, diglycerol, polyethylene glycol, triglycerol, tetraglycerol, polyglycerol), dihydric alcohol alkyl ethers (ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether), dihydric alcohol alkylethers (diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether), dihydric alcohol ether esters (ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate), glycerol monoalkyl ethers (chimyl alcohol, selachyl alcohol, batyl alcohol), sugar alcohols (sorbitol, maltitol, maltotriose, mannitol, saccharose, erythritol, glucose, fructose, starch decomposition sugar, maltose, xylitose, starch decomposition sugar reduction alcohol), glysolid, tetrahydrofurfuryl alcohol, POE tetrahydrofurfuryl alcohol, POP butyl ether, POP POE butyl ether, tripolyoxypropylene glycerol ether, POP glycerol ether, POP glycerol ether phosphate, POP POE pentaneerythritol ether, polyglycerol.

Monosaccharides:

Trioses (D-glyceryl aldehyde, dihydroxyacetone) tetroses (D-erythrose, D-erythrulose, D-threose, erythritol) pentoses (L-arabinose, D-xylose, L-lyxose, D-arabinose, D-ribose, D-ribulose, D-xylulose, L-xylulose) hexoses (D-glucose, D-talose, D-psicose, D-galactose, D-fructose, L-galactose, L-mannose, D-tagatose) heptoses (aldoheptose, heprose) octoses (octulose) deoxy sugars (2-deoxy-D-ribose, 6-deoxy-L-galactose, 6-deoxy-L-mannose) amino sugars (D-glucosamine, D-galactosamine, sialic acid, aminouronic acids, muramic acid) uronic acids (D-glucuronic acid, D-mannuronic acid, L-guluronic acid, D-galacturonic acid, L-iduronic acid)

Oligosaccharides:

Saccharose, gentianose, umbelliferose, lactose, planteose, isolignose, $\alpha,\alpha$-trehalose, raffinose, lignose, Umbilicine, stachyose, verbascose.

Polysaccharides:

Cellulose, quince seed, chondroitin sulfate, starch, galactan, dermatan sulfate, glycogen, gum arabic, heparan sulfate, hyaluronic acid, tragacanth gum, keratan sulfate, chondroitin, xanthan gum, mucoitin sulfate, guar gum, dextran, keratosulfate, locust bean gum, succinoglucan, charonic acid.

Amino acids:

Neutral amino acids (threonine, cysteine), basic amino acid (hydroxylysine), amino acid derivatives: sodium acylsarcosinates (sodium lauroylsarcosinate), acyl glutamates, acyl $\beta$-alanine sodium salts, glutathione, pyrrolidone carboxylates.

Organic amines:

Monoethanolamine, diethanolamine, triethanolamine, morpholine, triisopropanolamine, 2-amino-2-methyl-1,3-propanediol, 2-amino-2-methyl-1-propanol.

Macromolecule emulsions:

Acrylic resin emulsions, ethyl polyacrylate emulsions, acrylic resin liquids, polyacryl alkyl ester emulsions, polyvinyl resin acetate emulsions, natural rubber latex.

pH regulators:

Lactic acid - sodium lactate, citric acid - sodium citrate, succinic acid - sodium succinate.

Vitamins:

Vitamin A, vitamin B1, vitamin B2, vitamin B6, vitamin C, vitamin E, derivatives thereof, pantothenic acid and derivatives thereof, biotin.

Antioxidants:

Tocopherol, dibutylhydroxytoluene, butyl hydroxyanisol, gallate esters.

Antioxidant auxiliaries:

Phosphoric acid, citric acid, ascorbic acid, maleic acid, malonic acid, succinic acid, fumaric acid, cephalin, hexametaphosphates, phytic acid, ethylenediaminetetraacetic acid.

Other components that can be blended in:

Preservatives (ethylparaben, butylparaben), antiphlogistics (glycyrrhizinic acid derivatives, glycyrrhetic acid derivatives, salicylic acid derivatives, hinokitiol, zinc oxide, allantoin), whitening agents (placenta extract, saxifrage extract, arbutin), various extracts (Phellodendoron amurense, coptis, lithospermum, peony, Japanese swertia, birch, sage, loquat, carrot, aloe, mallow, iris, grape, coix seed, sponge gourd, lily, saffron, cnidium, zingiber, hypericum, restharrow, garlic, capsicum, citrus unshiu peel, Japanese angelica root, seaweed), activators (royal jelly, photosensitizer, cholesterol derivatives), blood circulation promoters (vanilyl amide nonylate, nicotinic acid benzyl ester, nicotinic acid β-butoxy ethyl ester, capsaicin, zingerone, cantharis tincture, ichthammol, tannic acid, α-borneol, tocopherol nicotinate, inositol hexanicotinate, cyclandelate, cinnarizine, tolazoline, acetylcholine, verapamil, cepharanthin, γ-oryzanol), anti-seborrheic agents (sulfur, thianthol), anti-inflammatory agents (tranexamic acid, thiotaurine, hypotaurine).

EXAMPLES

The present invention will now be described more specifically through examples. However, the present invention is not limited to only the following examples. In the following, unless otherwise stated, when contents are expressed in '%', this means mass%.

[Example 1]

The hair growth period extending action of N-methyltaurine was evaluated. Firstly, a description will be given of in vitro cell proliferation tests.

<Cell proliferation tests using cultured hair follicle epithelial cells>

A. Human hair follicle epithelial cells

1. Collection of human hair follicle epithelial cells

Hair follicles in the growth period of the hair cycle were collected mechanically under a stereoscopic microscope from male human scalp skin obtained as a by-product from surgery.

The growth period hair follicles were treated for 30 minutes at 37°C with Dulbecco's modified MEM (DMEM) containing 1000U/ml dispase and 0.2% collagenase, and the dermal sheath, dermal papilla and hair bulb epithelial tissue were removed using the tip of an injection needle, and then treatment was carried out for 5 minutes at 37°C with a phosphate buffer solution (PBS(-); '(-)' means not containing calcium ions, magnesium ions etc.) containing 0.05% trypsin and 0.02% EDTA.

Next, the hair follicles were left standing in a culture dish coated with collagen (type I), and explant culturing was carried out.

The culture medium used was a serum-free medium (keratinocyte growth medium (KGM)) (keratinocyte serum free medium can also be used).

After 4 to 5 days of culturing, when sticking of the hair follicles to the culture dish and cell proliferation were observed, the medium was exchanged, and then medium exchange was carried out every 2 days after this.

The cells thus proliferated were treated for 5 minutes at 37°C with 0.05wt% trypsin and 0.02% EDTA, the reaction was then stopped with an equivalent of 0.1% trypsin inhibitor, and then the cells were recovered by centrifuging (800×g, 5 minutes).

Next, the cells were suspended in the above-mentioned serum-free medium, sowing was carried out in a collagen-coated (type I) culture dish at a density of 5000cells/cm$^2$, medium exchange was carried out every 2 days until the cells became subconfluent, and then treatment was again carried out for 5 minutes at 37°C with 0.05wt% trypsin and 0.02% EDTA, the reaction was then stopped with an equivalent of 0.1% trypsin inhibitor, and centrifuging (800xg, 5 minutes) was carried out. A cell freezing liquid (Cellbanker; made by DAIYATRON) was added to the human hair follicle epithelial cells thus obtained, the concentration was adjusted to $1.0 \times 10^6$ cells/ml, $1.0 \times 10^6$ cells were put into each freezing tube, and storage was carried out by freezing. The number of cells was calculated using a haemacyto meter.

2. Assaying of object substance

The fibroblast contamination rate (FB contamination rate) of the hair follicle epithelial cells obtained through the above process was measured (3000x, 5 visual fields), and those for which the FB contamination rate was found to be 3% or more were excluded from the assaying.

The hair follicle epithelial cells were then sown into a culture flask, treatment was carried out with 0.05% trypsin and 0.02% EDTA, the reaction was stopped with 0.1% trypsin inhibitor, the system was centrifuged at 1500rpm for 5 minutes, the supernatant was removed, and 20ml of KGM medium was added to the residue, thus preparing a cell suspension.

Sowing was carried out in a 96-well plate (type I collagen-coated plate; made by Falcon) at a rate of 0.2 ml/well ($1.0 \times 10^4$ cells/well), and the plate was left standing for about 20 minutes at room temperature until the cells sank to the bottom of the wells. Culturing was then carried out for 1 day at 37°C with 5% $CO_2$, thus obtaining the desired cultured human hair follicle epithelial cells.

B. Rat hair follicle epithelial cells

1. Collection of rat hair follicle epithelial cells

(1) Collection of hair follicles

Dorsal skin was collected from newborn (3~ 4-day-old) rats, and this collected dorsal skin was soaked two pieces at a time in PBS (-) containing 1% PSF.

Lower parts such as subcutaneous fat and membranes were then removed from the dermal fat layer using dissecting scissors.

Next, the dorsal skin was again soaked in PBS (-) containing 1% PSF, and was then soaked overnight at 4°C in PBS (-) containing 0.25% trypsin (also containing 0.02% EDTA; likewise hereinafter).

After soaking in the trypsin solution, the corium layer and the epidermal layer were peeled off the dorsal skin using tweezers, and the corium layer was transferred to a 100mm dish into which had been put Ham's F12 medium [composition (mg./L) : 1-alanin (8.9), 1-arginine (HCl: 211), 1-asparagine (13.2), 1-asparatic acid (13.3), 1-cysteine (HCl: 31.5), 1-glutamic acid (14.7), 1-glutamine (146), glycine (7.5), 1-histidine (HCl: 19), 1-isoleucine (3.9), 1-leucine (13.1), 1-lysine (HCl: 36.5), 1-methionine (4.5), 1-phenylalanin (5.0), proline (34.5), 1-serine (10.5), 1-threonine (11.9), 1-tryptophane (2.0), 1-tyrosine (5.4), 1-valine (11.7), biotine (0.0073), choline(Cl: 14.0), vitamin B12 (1.36), folic acid (1.32), inositol (18.0), nicotinamide (0.037), pantothenic acid (Ca: 0.477), vitamin B6 (HCl: 0.062), vitamin B2 (0.038), vitamin B1 (HCl: 0.337), $CaCl_2$ ($2H_2O$: 44.0), $CuSO_4 \cdot 5H_2O$ (0.0025), $FeSO_4 \cdot 7H_2O$ (0. 834), KCl (224. 0), $MgCl_2$ ($6H_2O$: 122), "Proc. Natl. Acad. Sci. USA, 53, 288 (1965)"; likewise hereinafter] containing 0.35% collagenase, and was then cut with scissors. Infiltration was carried out on the medium containing the cut corium layer for 35 minutes at 37°C (60rpm).

After the infiltration, pipetting was carried out until lumps could no longer be seen in the collagenase reaction system, the system was transferred into a 50ml centrifuging tube, Ham's F12 medium containing Dnase (10000 units) was added, and the system was left standing for 5 minutes.

After leaving standing, the suspension obtained was again pipetted, and was then filtered using nylon mesh (Nytex 157 mesh), and transferred into a 50ml centrifuging tube. The suspension was divided into half, and each half was diluted with PBS (-) until the volume was 30ml, and then the diluted suspension was centrifuged (4°C, 400rpm, 5 minutes). After the centrifuging, the supernatant was taken off, thus removing the fat content from the system. Next, 25ml of PBS (-) was added to the residue to make a suspension, and then centrifuging was carried out again [(4°C, 400rpm, 5 minutes) × 3 times]. The residue obtained by this centrifuging procedure was the hair follicles from the rat dorsal skin.

(2) Collection of hair follicle epithelial cells

5 ml of PBS (-) containing 0.25% trypsin was added to the hair follicles obtained through the above procedure, and the cell suspension was then incubated for 5 minutes at 37°C.

After the incubation had been completed, a 5ml equivalent of fetal bovine serum (FBS) and Ham's F12 medium were added, and the cell suspension was filtered using a cell strainer (100μm, made by Nalgene), and was then put into a 50ml centrifuging tube, and centrifuged (4°C, 1500rpm, 5 minutes).

The supernatant was removed from the system, thus obtaining the desired hair follicle epithelial cells as the residue.

A cell freezing liquid (Cellbanker; made by DAIYATRON) was added to the hair follicle epithelial cells, the concentration was adjusted to $1.5 \times 10^7$ cells/ml, $1.5 \times 10^7$ cells were put into each freezing tube, and storage was carried out by freezing. The number of cells was calculated using a haemacyto meter.

2. Pre-culturing of hair follicle epithelial cells

To remove fibroblasts from the system as much as possible, pre-culturing of the hair follicle epithelial cells obtained through the above process was carried out.

Following is a description of the procedure used.

The frozen cells obtained through the above process were thawed in a 37°C constant temperature bath. Next, 10ml of an FAD medium [a mixture of Ham's F12 medium (described later) and an MEN medium in a volume ratio of 3: 1, containing insulin (5.0μg/ml), hydrocortisone (0.45μg/ml), epidermal growth factor (10.0ng/ml), cholera toxin ($10^{-9}$M) and fetal bovine serum (10%); likewise hereinafter] was added, the cell solution was diluted, and centrifuging was carried out (10°C or below, 1500rpm, 5 minutes). After the centrifuging, the supernatant was removed, 10ml of the FAD medium was added to the system, and pipetting was repeated until cell clusters could no longer be seen.

The number of cells obtained was calculated using a haemacyto meter, and adjustment was carried out with the FAD medium such that the concentration became $2.5 \times 10^5$ cells/ml. The cells were sown into a 75cm³ flask coated with type I collagen, and culturing was carried out overnight at 37°C with 5%CO₂.

After the culturing, the system was washed twice with 10ml of PBS (-), and then 2ml of PBS (-) containing 0.25% trypsin was added, and incubation was carried out for 4 minutes at 37°C with 5%CO₂. Next, 2ml of fetal bovine serum (FBS) was added to the system, the system was shaken once lightly, and then the supernatant was removed, thus removing fibroblasts contaminating the system.

15 ml of KGM medium [keratinocyte growth medium: keratinocyte basal medium {KBM medium (modified MCDB153 medium: made by Clonetics)} with bovine pituitary extract (BPE) (0.4vol%), insulin (0.5μm/ml) hydrocortisone (0.5μm/ml) and h-EGF (0.1ng/ml) added] was then added to the system, and culturing was carried out for 3 days at 37°C with 5%CO₂.

3. Assaying of object substance

The fibroblast contamination rate (FB contamination rate) of the culture flask in which the hair follicle epithelial cells obtained through the above process were sown was measured (3000x, 5 visual fields), and those for which the FB contamination rate was found to be 3% or more were excluded from the assaying.

The system was washed twice with 10ml of PBS (-), and then 2ml of PBS (-) containing 0.25% trypsin was added, and incubation was carried out for 3 minutes at 37°C.

Next, utilizing the difference in the reactivity of epithelial cells and fibroblasts toward trypsin, to remove the fibroblasts from the system, the trypsin was removed, and then 2ml of PBS (-) containing 0.25% trypsin was once again added, and shaking was carried out for 5 minutes at 20rpm and 37°C.

Next, after confirming peeling off of the cells under a microscope, 10ml of DMEM medium containing 10% FBS was added, pipetting was carried out in a 50ml centrifuging tube, and the system was centrifuged for 5 minutes at 1500rpm.

The supernatant was then removed, 20ml of KGM medium was added, and pipetting was carried out until cell clusters disappeared.

The suspension was filtered using a cell strainer (100μm, made by Nalgene), and was then put into a 50ml centrifuging tube, the number of living cells in the suspension was calculated using a haemacyto meter, and KGM medium was added to the system, and adjustment was carried out until the cell concentration in the system was $5.0 \times 10^4$cell/ml. Next, sowing was carried out in a 96-well plate (type I collagen-coated plate; made by Falcon) at a rate of 0.2 ml/well ($1.0 \times 10^4$ cells/well), and the plate was left standing for about 20 minutes at room temperature until the cells sank to the bottom of the wells. Culturing was then carried out for 1 day at 37°C with 5% CO₂, thus obtaining the desired cultured rat hair follicle epithelial cells.

C. Preparation of test media

(1) Preparation of medium with object substance added

About 1. 5mg of N-methyltaurine was weighed out, a 1% solution was prepared using KBM medium, and filtration sterilization was carried out using a 0.45$\mu$m filter.
Next, the above solution was added to 10,000 times the amount of KBM medium (object substance concentration: $1.0 \times 10^{-5}\%$).

(2) Preparation of control media

KBM medium was used as a negative control.
A medium in which 2$\mu$l of cell growth factor insulin (5mg/ml) and 2$\mu$l of hydrocortisone (0.5mg/ml) were added to the negative control KBM medium was used as a positive control.

D. Object substance medium exchange

The KGM media in the 96-well plates in which the cultured human hair follicle epithelial cells and the cultured rat hair follicle epithelial cells had been prepared in A and B above were exchanged with the object-substance-added medium and the control media (200$\mu$l/well), and after the exchange culturing was carried out for 2 days at 37°C with 5%$CO_2$.
The medium exchange was carried out as follows. The KGM medium in the wells was taken out with an aspirator while taking care not to damage the cells stuck to the bottom, and then immediately afterwards the object-substance-added medium was added from both edges of the wells.

E. Measurement of cell proliferation

One tenth the amount of alamar blue (made by Alamar Bioscience) was added relative to the amount (volume) of the medium, and incubation was carried out for 6 hours at 37°C (5%$CO_2$).
After the incubation, the absorbance of the system at 595nm and 570nm was measured using a microplate reader (made by Bio RAD), and the extent of cell proliferation was calculated from the following calculation formula.

$$\text{(Cell proliferation extent for object sample)} =$$
$$\text{(Alamar blue reduction rate for object sample)} /$$
$$\text{(Alamar blue reduction rate for negative control)} \times$$
$$100 \ (\%)$$

Moreover, the hair follicle epithelial cell proliferation effect of N-methyltaurine was judged from the following calculation formula.

$$(Cell\ proliferation\ index\ for\ object\ sample) = ((Cell\ proliferation\ extent\ for\ object\ sample) - (Alamar\ blue\ reduction\ rate\ for\ negative\ control)) / ((Alamar\ blue\ reduction\ rate\ for\ positive\ control) - (Alamar\ blue\ reduction\ rate\ for\ negative\ control))$$

[Results]

The cell proliferation effect of N-methyltaurine determined as above was 0.64 for the cultured human hair follicle epithelial cells, and 0.62 for the cultured rat hair follicle epithelial cells.

It is 0 for the negative control, and 1 for the positive control.

From these results, it can be seen that N-methyltaurine was clearly found to have an excellent cultured hair follicle epithelial cell proliferation activity. That is, it is clear that N-methyltaurine has a hair growth period extending activity.

[Example 2]

The immortalized outer hair root sheath cell proliferation action of N-methyltaurine was evaluated. Firstly, a description will be given of immortalized outer hair root sheath cell proliferation tests.

<Immortalized outer hair root sheath cell proliferation tests>

[Culturing of immortalized outer hair root sheath cells]

Hair follicles were isolated from human scalp skin using scissors under a stereoscopic microscope. The hair follicles were cut off at the lower part of the sebaceous gland, and enzyme treatment was carried out with collagenase and dispase.

The hair bulb was cut off with scissors and excluded, and the hair shaft was separated with tweezers.

The hair shaft was subjected to enzyme treatment with trypsin, and then the reaction was stopped with trypsin inhibitor.

Centrifuging was carried out, the supernatant was thrown away, and the outer hair root sheath cells were recovered.

The recovered cells were sown with keratinocyte growth medium (KGM) into a collagen-coated culturing flask, and culturing was carried out in a $CO_2$ incubator.

Virus and transgene

A virus for which the E1A region of $\Delta$ E1/X, which is an adenovirus vector, had been replaced with a large T antigen gene of SV40 with the replication origin deleted was used (Doren and Gluzman, 1984; Mol. Cell. Biol. 4, 1653-1656).

Introduction of T antigen gene

First generation cultured outer hair root sheath cells cultured until the cloning confluence of the cells was about 50% were washed with K-SFM, and then infection was carried out by adding the above-mentioned virus in a quantity of 1, 10 or 30 MOI (multiplicity of infection).

Subsequently, passage culturing was continued as with normal cells, and cloning was carried out after the passage number at which proliferation of normal cells would stop had been reached.

In the cloning, $10^3 \sim 10^4$ cells only were re-sown per 10cm-diameter petri dish, cells for which proliferation was good and the form was unchanged from that of normal cells were picked up using the tip of a Pipetman, these were transferred into a 24-well plate and cultured, and cells for which proliferation was good even at this time were selected.

Note that passage culturing was also continued as with normal cells on the selected cell line.

The results were that the outer hair root sheath cells not infected with the virus stopped proliferating after about 5 generations.

The T-antigen-introduced hair papilla cells appeared to have stopped proliferating about 7 generations after cloning, but when culturing was continued, they appeared to start proliferating again.

It was surmised that that a crisis probably occurred at the seventh generation, and at this time some kind of mutation occurred, and the cells became immortalized. During the cloning, a number of clones were selected, but one cell line clone was obtained for which the crisis period was passed and proliferation continued.

Evaluation of cell proliferation

The outer hair root sheath cells were washed twice with PBS (-). Enzyme treatment with trypsin was carried out, thus peeling off the cells.

The reaction was stopped with trypsin inhibitor, centrifuging was carried out, the supernatant was thrown away, and the outer hair root sheath cells were recovered. KGM medium was added, thus preparing a cell suspension.

The cells were sown in a 24-well culture plate coated with collagen, and culturing was carried out in a $CO_2$ incubator.

The next day, the medium was exchanged with a medium to which the test substance had been added. After culturing for 4 days, the cells were washed with PBS (-), and then the cells were peeled off with trypsin. The cells were frozen plate by plate in this state.

Preparation of test substance

The test substance N-methyltaurine was prepared to 50mM using keratinocyte basal medium (KBM), and filtration sterilization was carried out. Taking this as a stock solution, dilution with the KBM medium was carried out, thus making the test substance concentration 10nM, 1μM, 100μM or 10mM. KBM medium only was taken as a negative control.

Cellular DNA measurement

The cells were thawed, and were then added to each hole of a Hoechst 33258, and smashed up with sonication. The cells were then transferred into a cuvette, and the fluorescence intensity was measured at an excitation wavelength of 356nm and a fluorescence wavelength of 460nm. Taking the fluorescence intensity to be 100 for the negative control, the relative DNA amount was calculated, and hence the extent of cell proliferation was calculated.

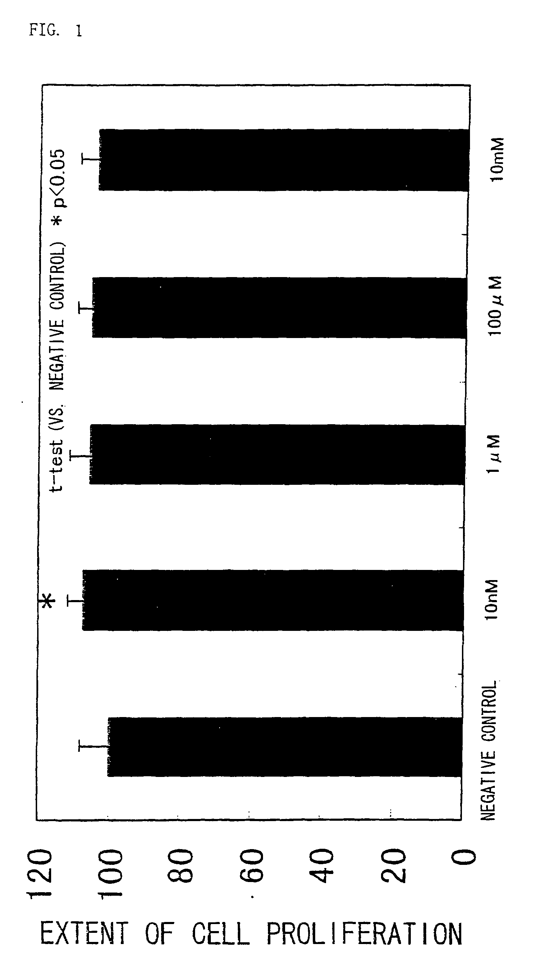

The results are shown in Fig. 1. From the results, it can be seen that N-methyltaurine has an immortalized outer hair root sheath cell activation action.

Next, the hair fostering effects based on the hair growth period extending action will be examined.

[Example 3] Liquid hair growth agent

0.8% of N-methyltaurine was mixed with agitation with 90% of 70% ethanol, 0.05% of sodium oleate, 0.49% of dodecylbenzenesulfonic acid, 0.5% of hardened castor oil ethylene oxide (40mol) adduct, and ion exchange water (the remainder), thus carrying out dissolution.

Ion exchange water (10%) was then further added and mixing was carried out, thus obtaining a liquid hair growth agent. As a control, a liquid agent was also prepared using this formulation but without the N-methyltaurine (Comparative Example 1).

[Example 4] Milky lotion type hair growth agent (Reference)

A milky lotion type hair growth agent of the following formulation was prepared.

| Component | Content (mass%) |
|---|---|
| (A phase) | |
| N-methyltaurine | 0. 05 |
| Polyoxyethylene(60 mol)-added hardened castor oil | 2. 0 |
| Glycerol | 10. 0 |
| Dipropylene glycol | 10. 0 |
| 1,3-butylene glycol | 5. 0 |
| Polyethylene glycol 1500 | 5. 0 |

(continued)

| Component | Content (mass%) |
|---|---|
| (B phase) | |
| Cetyl isooctanoate | 10. 0 |
| Squalane | 5. 0 |
| Vaseline | 2. 0 |
| Propylparaben | 2. 0 |
| (C phase) | |
| Carboxyvinyl polymer 1% aqueous solution | 30.0 |
| Sodium hexametaphosphate | 0. 03 |
| Ion exchange water | 9. 3 |
| (D phase) | |
| Ion exchange water | 4. 5 |
| (E phase) | |
| KOH | 0.12 |
| Ion exchange water | 5. 0 |

<Manufacturing method> The A phase and the B phase were each heated and dissolved at 60°C, and were then mixed together using a homomixer to produce a gel. The D phase was then gradually added thereto, and dispersion was carried out in the homomixer. The dissolved C phase was next added, and then finally the dissolved E phase was added, and emulsification was carried out in the homomixer, thus preparing an O/W milky lotion type hair growth agent.

[Example 5] Cream type hair growth agent (Reference)

| Component | Content (mass%) |
|---|---|
| (A phase) | |
| Liquid paraffin | 5. 0 |
| Ceto-stearyl alcohol | 5. 5 |
| Glyceryl monostearate | 3. 0 |
| EO(20 mol)-2-octyldodecyl ether | 8. 0 |
| Propylparaben | 0. 3 |
| Aromatics | 0.1 |
| (B phase) | |
| N-methyltaurine | 5. 0 |
| Glycerol | 8. 0 |
| Dipropylene glycol | 20. 0 |
| Polyethylene glycol 4000 | 5. 0 |
| Sodium dodecyl sulfate | 0. 1 |
| Sodium hexametaphosphate | 0. 005 |
| Ion exchange water | 39. 995 |

<Manufacturing method> The A phase and the B phase were each heated and dissolved, mixed together, and then emulsification was carried out in a homomixer, thus obtaining a cream type hair growth agent.

[Examination of hair fostering action of hair growth agents]

To investigate the hair fostering action such as hair loss prevention and hair growth effects of the hair growth agents obtained above, trichogram tests and actual usage tests were carried out on humans using the following method. The test samples were the hair growth agents of Examples 3~5, 70% ethanol, and Comparative Example 1.

Test method

The roots of extracted hairs were observed under a microscope before and after usage of the above mentioned samples, the proportion of 'resting period hairs' for which the hair had stopped growing was calculated from the form of the hair root, and the hair fostering actions of the samples were compared by means of increases/decreases in this proportion.

Specifically, each test sample was applied continuously twice a day for 6 months onto the scalps of 10 male testees, with 2ml being applied each time; 100 hairs were extracted from each testee both immediately before the application and immediately after the 6 months of application had been completed, and each hair root was observed under a microscope. The results of the test are shown in 'Table 1' below.

Table 1

| Sample (control I and hair growth agent number) | Proportion of resting period hair roots | | | Evaluation of hair fostering effect |
|---|---|---|---|---|
| | Reduction of 20% or more (%) | $\pm$ 20% (%) | Increase of 20% or more (%) | |
| Control (70% ethanol) | 10 | 50 | 40 | Ineffective |
| Example 3 | 50 | 40 | 10 | Effective |
| Example 4 | 40 | 40 | 20 | Effective |
| Example 5 | 60 | 30 | 10 | Effective |
| Comparative Example 1 | 20 | 40 | 40 | Ineffective |

From these results, it was found that the hair growth agents of the present invention have a hair fostering effect based on a hair growth period extending effect.

[Example 6: Hair fitness/body improving agent] (Reference)

The effects of a hair fitness/body improving agent comprising N-methyltaurine were evaluated. Firstly, a description will be given of the test method.

Test method

The hair of a 19-year-old woman with no history of any chemical treatment such as perming, coloring or bleaching was used. About 20cm of the hair tip part was immersed in a prescribed shampoo solution for 1 hour, washed for 1 minute under running water, and dried for more than 24 hours in a normal environment; this was taken as a normal healthy hair sample. This normal healthy hair was bleached for 30 minutes at room temperature using a prescribed bleaching agent, and was then washed for 1 minute under running water. This bleaching treatment was repeated 4 times, and after washing, the hair was dried in a normal environment; this was then taken as bleached (BL-treated) hair.

N-methyltaurine treatment

A single strand of hair was immersed overnight in 20ml of a 1mol/l N-methyltaurine aqueous solution, and then the test sample hair was dried under a 25°C 50%RH environment.

Measurement of torsional torque

Measurements were taken under a 25°C 50%RH environment using a KES-YN-1 torsion test apparatus made by Kato Tech. Measurements were taken before treatment with the N-methyltaurine aqueous solution, with this being taken as a control. The twisting angle was $\pm$1080°, and twisting was carried out at a rate of 18°/sec.

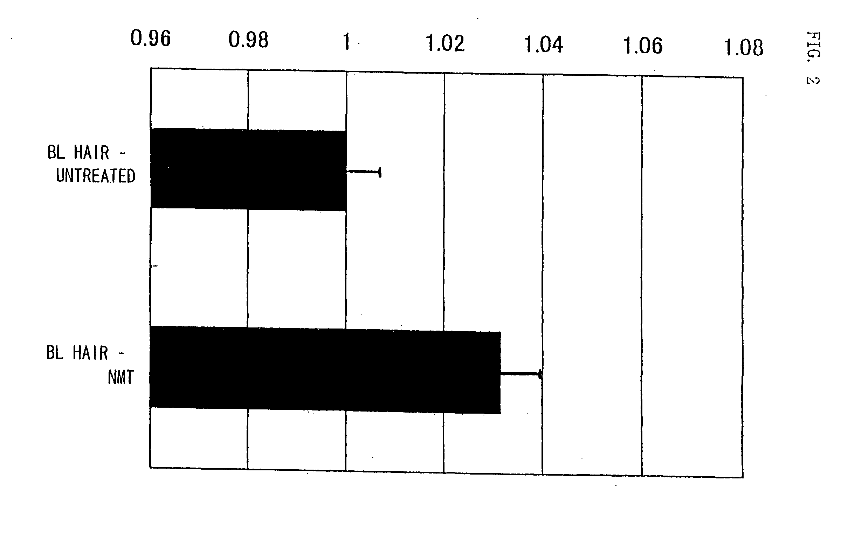

For a twisting angle $\theta = 360°\sim720°$, $B = \tan(Tf/\theta)$, which is the increase in the torsional torque $Tf$ with the twisting angle $\theta$, was taken as the torsional rigidity $B$, and evaluation was carried out using the ratio of the $B$ values before and after the treatment with the N-methyltaurine aqueous solution. The results are shown in Fig. 2.

From the results, it can be seen that the torsional torque is increased for the hair treated with N-methyltaurine, and hence N-methyltaurine gives hair fitness and body.

Other examples of the present invention are given below.

[Example 7] Hair fostering shampoo

| Component | Content (mass%) |
|---|---|
| N-methyltaurine | 8. 0 |
| Polyoxyethylene alkyl ammonium sulfate | (2E.0) |
| | 15. 0 |
| Sodium amidopropyldimethylacetate | 3. 0 |
| Coconut oil fatty acid monoethanolamide | 1. 6 |
| Ethylene glycol distearate | 0. 6 |
| Dimethyl silicone (5000cs) emulsion 40% solution | |
| | 1. 8 |
| Sodium benzoate | 0. 2 |
| Cationized cellulose | 0. 3 |
| Ion exchange water | 69.5 |

[Example 8] Hair fostering rinse

| Component | Content (mass%) |
|---|---|
| N-methyltaurine | 0. 6 |
| Cyclomethicone | 3. 4 |
| Dimethyl silicone (1,000,000 mPa·s) | 0. 5 |
| Stearyl alcohol | 7. 5 |
| Stearic acid dimethylaminopropylamide | 2. 5 |
| Ion exchange water | 85. 5 |

INDUSTRIAL APPLICABILITY

Effects of the hair growth agent of the present invention are as follows.

(1) The hair growth agent of the present invention activates hair follicle epithelial cell proliferation, and thus extends the growth period in the hair cycle.
(2) The hair growth agent of the present invention gives hair fitness and body, and gives hair a feeling of volume.

Moreover, by combining N-methyltaurine with an existing cleaning component such as an acylmethyltaurine type cleaning component, a hair cleaning agent that has high stability and enables N-methyltaurine to be adsorbed into hair follicles and hair purely by shampooing can be provided.
The Reference Examples are not part of the invention.

**Claims**

1. A hair fostering shampoo or rinse containing N-methyltaurine.

**Patentansprüche**

1. Haarpflegeshampoo oder -spülung, enthaltend N-Methyltaurin.

**Revendications**

1. Shampoo ou rinçage pour le soin des cheveux contenant du N-methyltaurine.

FIG. 1

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Proc. Natl. Acad. Sci. USA,* 1965, vol. 53, 288

- **DOREN ; GLUZMAN.** *Mol. Cell. Biol.,* 1984, vol. 4, 1653-1656